(19) Europäisches Patentamt
European Patent Office
Office européen des brevets

(11) **EP 3 524 306 B1**

(12) **EUROPÄISCHE PATENTSCHRIFT**

(45) Veröffentlichungstag und Bekanntmachung des Hinweises auf die Patenterteilung:
**04.08.2021 Patentblatt 2021/31**

(51) Int Cl.:
*A61M 16/00* *(2006.01)*       *A61B 5/085* *(2006.01)*
*A61B 5/087* *(2006.01)*       *A61B 5/00* *(2006.01)*
*A61B 5/113* *(2006.01)*

(21) Anmeldenummer: **19156708.0**

(22) Anmeldetag: **12.02.2019**

(54) **BEATMUNGSGERÄT SOWIE TESTVERFAHREN**

ARTIFICIAL RESPIRATION DEVICE AND TEST METHOD

APPAREIL RESPIRATOIRE AINSI QUE PROCÉDÉ D'ESSAI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.02.2018 EP 18156357**

(43) Veröffentlichungstag der Anmeldung:
**14.08.2019 Patentblatt 2019/33**

(73) Patentinhaber: **Fritz Stephan GmbH**
**56412 Gackenbach (DE)**

(72) Erfinder:
• **Höhne, Bernd**
**56130 Bad Ems (DE)**
• **Braun, Wolfgang**
**56428 Dernbach (DE)**

(74) Vertreter: **Hellmich, Wolfgang**
**European Patent and Trademark Attorney**
**Lortzingstrasse 9 / 2. Stock**
**81241 München (DE)**

(56) Entgegenhaltungen:
**WO-A1-2011/117787       WO-A1-2013/126417**
**WO-A1-2018/006121       DE-A1- 19 617 432**
**DE-A1-102007 035 549     DE-A1-102012 014 141**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung bezieht sich auf ein Beatmungsgerät und ein Steuer- und Testverfahren, bei dem für die Wechsel zwischen Inspirations- und Exspirationsmodus sowohl Atemfluss als auch ein Abdomenbewegungssignal berücksichtigt werden.

Beatmungsgeräte blasen während einer Inspirationsphase Luft in die Lungen eines Patienten und lassen die Luft während einer Exspirationsphase in die Umgebung entweichen. Der während der Inspirationsphase benötigte Überdruck kann im Beatmungsgerät über ein regelbares Gebläse zur Verfügung gestellt werden. Üblicherweise ist in diesem Fall ein Drucksensor nach dem Gebläse angeordnet und eine gegenüber der Atmung schnelle Regelschleife regelt die Gebläsedrehzahl so, dass der vom Drucksensor gemessene Druck einem eingestellten Solldruck entspricht.

Alternativ kann ein Beatmungsgerät den benötigten Überdruck auch aus einer Krankenhausgasversorgung oder einer Gasflasche beziehen. In diesem Fall kann ein Proportionalventil vorhanden sein, das einen vorgegebenen Luftfluss einstellt, der mit einem Flusssensor gemessen wird. Eine im Vergleich zur Atmung schnelle Regelschleife regelt die Öffnung des Proportionalventils so, dass der gemessene Fluss einem eingestellten Fluss entspricht.

Die bisher aufgeführten Größen, nämlich Druck nach dem Gebläse, Gebläsedrehzahl, Öffnung des Proportionalventils, Fluss und Ähnliches werden in diesem Dokument unter dem Oberbegriff Beatmungsintensität zusammengefasst.

Beatmungsgeräte gemäß dem Stand der Technik weisen einen eingebauten Computer mit den typischen Elementen Prozessor, Arbeitsspeicher und nichtflüchtiger Massenspeicher auf. Solche Beatmungsgeräte weisen ferner einen oder mehrere Druck- und Flusssensoren, Anschlüsse für Beatmungsschläuche und vieles mehr auf. Bei solchen Beatmungsgeräten können verschiedene Beatmungsformen ausgewählt und vom Prozessor gesteuert durchgeführt werden.

Die nichtinvasive Beatmung ist besonders schonend und wird deshalb bevorzugt angewendet. Bei der nichtinvasiven Beatmung (Englisch: non-invasive ventilation, NIV), wird das Atemgas nicht über einen in die Luftröhre eingeführten Schlauch, den Tubus, in die Lunge geleitet, sondern über kurze Kanülen (Englisch: prongs), die auch als nasale Kanülen oder Nasenkanülen bezeichnet werden, in die Nase oder über eine Maske über Mund und/oder Nase appliziert. Die DE 11 2010 001 519 B4 beschreibt eine als atraumatischen Nasentubus bezeichnete Halterung mit Nasenstutzen, also kurze Kanülen, durch die Atemgas in die Nase appliziert wird. Der Nasentubus ist für Zweischlauchbeatmungssysteme mit Ein- und Ausatemschlauch (Anschlüsse 3 bzw. 4 in Fig. 1-5 der DE 11 2010 001 519 B4) gedacht. Insbesondere die Verwendung von Nasenkanülen führt zu hohen Leckflüssen, weshalb das Beatmungsgerät die ausgeatmete Luft nur sehr ungenau erfassen und somit die Wechsel zwischen Inspiration und Exspiration schlecht aufgrund der Gasflüsse im Inspirations- und Exspirationsschlauch ermitteln kann.

Außerdem ändern sich die Leckflüsse insbesondere bei der Beatmung von Neu- und Frühgeborenen stark und können den Volumenstrom der Einatmung bei weitem übertreffen. Bei der Beatmung sehr kleiner Kinder fließt nämlich durch das Beatmungsschlauchsystem ein permanenter Gasstrom, der wesentlich höher ist, als der zur Beatmung benötigte Gasstrom.

Eine Möglichkeit der Leckkorrektur ist in DE 10 023 473 A1 beschrieben. Hierbei wird aus dem mittleren Volumenstrom und dem mittleren Beatmungsdruck ein mittlerer Leckleitwert berechnet. Der eingeatmete Volumenstrom ergibt sich aus dem gemessenen Volumenstrom abzüglich des Produkts aus dem mittleren Leitwert und dem gemessenen Druck. Dieses Verfahren hat den Nachteil, dass plötzlich auftretende Leckveränderungen die Steuerung des Beatmungsgerätes stören.

In der DE 10 2012 014 141 A1 wird vorgeschlagen, insbesondere zum Detektieren des Beginns der Inspiration die radial exzentrische Abdomenbewegung zu erfassen. Die Detektiereinrichtung weist hierfür ein volumenveränderbares Fluidkammerelement mit einem durch eine radial exzentrische Abdomenbewegung beeinflussbares Fluidraumvolumen auf. Die Detektiereinrichtung weist ferner eine Befestigungseinrichtung auf, welche zirkular um den Abdomenbereich derart herum geschlungen ist, dass die Detektiereinrichtung ventral an dem Patienten angeordnet werden kann. Das Fluidkammerelement ist als ein elastisch verformbares und sich in sein Ausgangsfluidraumvolumen selbstständig zurückstellendes, schlauchartiges Fluidkissenteil mit einem elastischen Wabenstrukturelement ausgestaltet. An einem Anschlussteil ist eine Flussmesseinrichtung (Pneumotachograph) mit einem pneumatischen Widerstand befestigt, an der Druckschläuche angeschlossen sind, um den Differenzdruck an das Beatmungsgerät zu übermitteln.

Um dieses Verfahren zu realisieren ist es außerdem erforderlich, eine Referenzmessung an jedem Patienten durchzuführen, bei der sowohl der Atemfluss als auch der Druckabfall am pneumatischen Widerstand, also der Fluss in oder aus dem Fluidkammerelement, an mehreren Stellen in einem Atemzyklus gemessen wird, um später während der Beatmung aus dem Druckabfall den Atemfluss zu berechnen. Dies führt zu einem erheblichen Mehraufwand in der Anwendung.

Die DE 10 2007 035549 A1 offenbart ein Gerät zur Schlafüberwachung eines Patienten. Es umfasst eine zentrale Steuer- und Auswerteeinheit, an die zwei Dehnungssensoren in Form eines Thorax-Dehnungsmessgurts und eines Abdomen-Dehnungsmessgurts angeschlossen sind. Der Thorax-Dehnungsmessgurt wird im Bereich des Brustkorbs des Patienten und der Abdomen-Dehnungsmessgurt im Bereich des Bauchs des Patienten angelegt. Beide Messgurte erfassen die Atmungsbewegungen des Patienten. Der Thorax- und der Abdomen-Dehnungsmessgurt sind als Dehnungsmessstreifen oder Induktionsdruckgurte ausgeführt. Das Gerät umfasst weiterhin einen Flusssensor zur Erfassung der Atemluftventilation der an die Auswerte- und Steuereinheit anschließbar ist. Der Flusssensor ist insbesondere Teil der Subeinheit zur Atmungsunterstützung. Letztere umfasst außerdem eine Atemmaske und/oder zumindest einen Schlauch zur unterstützenden Luft- oder Sauerstoffzuführung. Bei Bedarf kann das Gerät auch steuernd zur Behebung einer erkannten Atmungsstörung eingreifen, indem ein Beatmungsdruck angehoben wird. Die Steuer- und Auswerteeinheit ermittelt eine zwischen dem Abdomen-Messsignal und dem Thorax-Messsignal auftretende Phasendifferenz.

Die EP 2 671 509 A1 offenbart ein ähnliches Respirations-Sensorelement mit einem Sensorteil, welches direkt auf die Haut seitlich am Abdomen (Bauch) des Patienten mittels medizinischen Klebebands appliziert wird. Das Sensorelement hat ferner ein Kabelteil, mittels welchem von dem Sensorteil erzeugte elektrische Signale an eine Datenschnittstelle einer Elektronikeinrichtung des Beatmungssystems übertragen werden. Die physikalische Messgröße scheint der Druck im Sensorteil zu sein. Die erzeugten Signale werden durch Bilden eines arithmetischen Mittelwertes in Bezug auf zuvor erzeugte Signale korrigiert und so kalibriert. Für den Fall, dass eine solche rechnerische Kalibrierung ausgeschlossen ist, umfasst das Beatmungssystem ein Ventilelement zur Belüftung und/oder Entlüftung des Beatmungssystems. Sowohl das gemäß der DE 10 2012 014 141 A1 als auch das gemäß der EP 2 671 509 A1 ermittelte Abdomenbewegungssignal weist einen nichtlinearen Zusammenhang mit dem Atemflusssignal auf.

Im Bereich der invasiven Beatmung von Neu- und Frühgeborenen werden Volumenstromsensoren meistens patientennah zwischen dem Y-Stück des Beatmungsschlauchsystems und dem Tubus oder Tubusadapter angebracht. Außerdem verbieten Größe und Gewicht der Sensoren diese Anordnung bei der nichtinvasiven Beatmung von Neu- oder Frühgeborenen.

Von der Erwachsenen-Beatmung ist die Verwendung patientenferner Volumenstromsensoren bekannt. Diese werden bei der Beatmung von Früh- und Neugeborenen kaum eingesetzt, auch weil bei der Beatmung sehr kleiner Kinder durch das Beatmungsschlauchsystem ein permanenter Gasstrom fließt, der wesentlich höher ist, als der zur Beatmung benötigte Gasstrom.

In DE 196 17 432 A1 wird eine Anordnung zur Messung des Volumenstroms beschrieben, bei welcher zwei Strömungselemente patientenfern, also am Beatmungsgerät angebracht sind. Beide Strömungselemente wandeln den Volumenstrom in eine Druckdifferenz und sind pneumatisch gekoppelt, damit nur ein Differenzdrucksensor zur Ausgabe eines elektrischen Signals benötigt wird. Dieses Verfahren liefert aber nur dann hinreichend genaue Messwerte, wenn die beiden Strömungselemente eine hinreichend gleiche Kennlinie aufweisen, also hinreichend gleiche Differenzdrücke bei gleichen Volumenströmen liefern.

Eine hinreichend gleiche Kennlinie weisen Strömungselemente auf, die über dünne Folien verfügen, die durch einen offenen Spalt voneinander getrennt sind. Eine Folie öffnet sich durch den Volumenstrom leicht, wodurch der Spalt vergrößert wird und der Widerstand mit steigendem Volumenstrom abnimmt. So vertreibt die Hamilton Medical AG (www.hamilton-medical.com) einen "infant Flowsensor", also einen Flusssensor zur Beatmung von Neugeborenen, der zwischen das Y-Stück und die Patientenschnittstelle, also z. B. einen Tubus, eingebaut wird. Die Öffnung öffnet sich mit steigendem Fluss weiter, so dass der differentielle Flusswiderstand von 12 mbar • s / l bei wenig Fluss bis auf 4,3 mbar • s / l bei einem Fluss von 0,5 l/s abfällt. Die DE 10 2010 000 219 A1 offenbart ebenfalls Strömungselemente, die als Durchflusssensoren bezeichnet werden. Diese Durchflusssensoren umfassen einen Ventilsitz und eine Ventilklappe aus einem elastomeren Material. Die Ventilklappe wird durch eine Vorspannkraft gegen den Ventilsitz gedrückt. Trotzdem soll die Durchflussrate proportional der Druckdifferenz zwischen den beiden Seiten der Klappe sein.

Die DE 699 24 163 T2 (Übersetzung des europäischen Patents EP 1 382 294 B1) beschreibt ein respiratorisches Unterstützungssystem. Zur Messung der EMG-Aktivität des Zwerchfells (EMGdi) eines menschlichen Patienten wird eine Anordnung von Elektroden am freien Endteil eines Ösophaguskatheters montiert. Der Katheter wird durch ein Nasenloch oder den Mund in den Ösophagus (Speiseröhre) des Patienten eingeführt, bis die Elektroden auf dem Niveau des gastroösophagealen Übergangs liegen. Generell sind Verfahren zur Erfassung der Zwerchfellaktivität mit einem erheblichen zusätzlichen apparativen Aufwand verbunden.

Die DE 699 22 511 T2 offenbart ebenfalls eine Steuerung eines Beatmungsgeräts durch neuroelektrische Signale. Ein Respirationsdetektor ist an den Nervus phrenicus entweder transkutan oder mittelbar über die Oberfläche der Haut aus angeschlossen. Diese Respirationssignale sind das früheste Anzeichen dafür, dass der Patient atmen möchte. Eine Analyse der Nervenimpulse kann laut DE 699 22 511 T2 u.a. mit Mustererkennungssystemen und künstlichen neuronalen Netzwerken (KNN) erfolgen. Nun ist allerdings der Nervus phrenicus ein sensomotorischer Nerv. Soweit der Erfinder weiß, gelang die Unterscheidung zwischen motorischen und sensorischen Signalteilen nie.

PAV (Proportional Assist Ventilation) ist eine populäre Beatmungsform, in der das Beatmungsgerät einen bestimmten Anteil der für die Atemtätigkeit notwendigen Arbeit übernimmt. Das Beatmungsgerät verändert das Atemvolumen und den Druck in Abhängigkeit der Atemtätigkeit des Patienten. Je stärker der Patient atmet, desto mehr Atemvolumen und Druck liefert das Beatmungsgerät. Es wird davon ausgegangen, dass mit zunehmender Atemtiefe die zu überwindenden Kräfte ansteigen und der Patient mehr Atemarbeit aufzubringen hat, was dazu führt, dass der Patient schneller ermüdet.

Die DE 40 38 871 A1 offenbart dass die begrenzenden Faktoren seitens der Atemmechanik für eine Langzeit-Eigenatmung frühgeborene Kinder sind stark erhöhte Atemwegswiderstände (Resistance) und eine verminderte Lungendehnbarkeit (Compliance), wobei meist die verminderte Compliance der dominierend krankhaft veränderte Parameter. den Beatmungsdruck P in Abhängigkeit vom Volumen V und/oder dem Volumenstrom V' zu wählen:

$$P = CPAP + fc(V) + fr(V') \qquad (1)$$

CPAP ist vermutlich ein konstanter Druck. Im einfachsten Fall sind fc und fr lineare Funktionen, so dass sich ergibt:

$$P = CPAP + Kc*V + Kr*V' \qquad (2)$$

Obwohl die DE 40 38 871 A1 PAV nicht explizit erwähnt, stellt insbesondere Gleichung (2) eine Form der PAV dar. Die Compliance C ist das eingeatmete Luftvolumen Vt dividiert durch den benötigten Druckunterschied $\Delta$P:

$$C = V_t / \Delta P \qquad (3)$$

Es ist Aufgabe der Erfindung ein Beatmungsgerät und ein Steuer- und Testverfahren anzugeben, das die Übergänge zwischen Exspiration und Inspiration auch bei sich verändernden Lecks zuverlässig erkennt.

Diese Aufgabe wird durch die Lehre der unabhängigen Ansprüche gelöst.

Bevorzugte Ausführungsformen der Erfindung sind Gegenstand der Unteransprüche.

Im Folgenden werden bevorzugte Ausführungsformen der Erfindung unter Bezugnahme auf die beiliegenden Zeichnungen näher erläutert. Dabei zeigen:

Fig. 1  eine mögliche zukünftig realisierte Ausführungsform, und

Fig. 2  eine aktuell realisierte Ausführungsform.

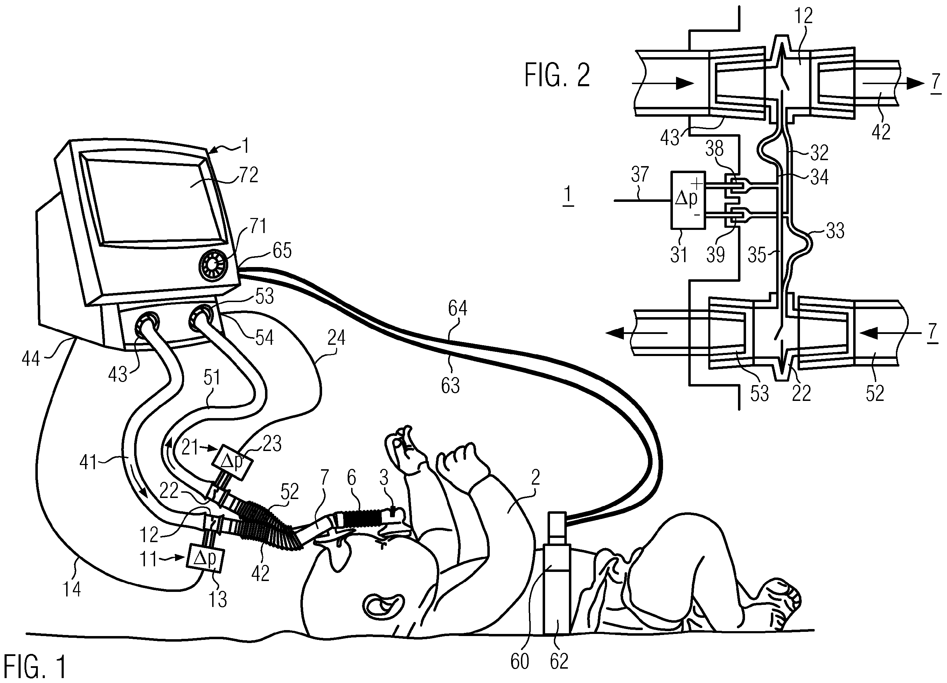

Figur 1 zeigt ein Beatmungsgerät 1 mit dem ein neonataler oder pädiatrischer Patient 2 beatmet wird. Anstelle des Patienten kann das Beatmungsgerät allerdings auch an ein Testgerät evtl. mit einem Kunstkopf angeschlossen sein, das die Atemtätigkeit eines Patienten lediglich simuliert. Das Beatmungsgerät weist einen Bildschirm 72 und ein Bedienelement 71 auf. Das Beatmungsgerät 1 liefert Frischluft über einen Einatemschlauchanschluss 43, einen ersten Einatemschlauch 41, einen Flusssensor 11, einen zweiten Einatemschlauch 42, ein Y-Stück 7, einen Beatmungsschlauch 6 sowie eine Nasenmaske 7 zum Patienten 2.

Ausgeatmetes Gas wird von der Nasenmaske 3 über den Beatmungsschlauch 6, das Y-Stück 7, einen ersten Ausatemschlauch 52, einen Flusssensor 21, einen zweiten Ausatemschlauch 51 und einen Ausatemschlauchanschluss 53 zum Beatmungsgerät 1 geleitet. Im Beatmungsgerät 1 ist meist ein Ventil, insbesondere ein Proportionalventil vorgesehen, über das das ausgeatmete Gas in die Umgebung abgegeben wird. Das Beatmungsgerät 1 weist einen eingebetteten Computer mit Prozessor und nichtflüchtigem Massenspeicher auf, der die Beatmung steuert. Über das Bedienelement 71 können unterschiedliche Beatmungsformen einschließlich PAV ausgewählt werden.

Jeder der Flusssensoren 11 und 21 besteht aus einem Strömungselement 12 bzw. 22 wie es oben beschrieben wurde und beispielsweise von der Hamilton Medical AG vertrieben wird. Im Hinblick auf die geforderte Linearität sei angemerkt, dass bei der Beatmung von neonataler oder pädiatrischen Patienten der Luftfluss in den Beatmungsschläuchen 41, 42, 51 und 52 deutlich größer als der eingeatmete und ausgeatmete Luftfluss des Patienten 2 ist. Deshalb schwankt der Luftfluss nur in einem kleinen Bereich der Kennlinie der Strömungselemente, sodass die Strömungselemente in diesem Bereich hinreichend linear sind. Die Druckdifferenz an jedem der Strömungselemente 12 bzw. 22 wird über einen über kurze Schläuche angeschlossenen Differenzdrucksensor 13 bzw. 23 in ein elektrisches Signal gewandelt, das über je eine elektrische Leitung 14 bzw. 24 und elektrische Anschlüsse 44 bzw. 54 an das Beatmungsgerät 1 übertragen wird. In anderen Ausführungsformen können die Differenzdrucksensoren 13 und 23 im Beatmungsgerät 1 untergebracht sein oder in die Strömungselemente 12 bzw. 22 integriert sein.

Die Differenzdrucksensoren 13 und 23 können entweder ein analoges oder bereits ein digitales Signal liefern. In anderen Ausführungsformen können auch Strömungselemente, die z.B. anstelle der Folien eine flexible Scheibe wie in der DE 10 2010 000 219 A1 als pneumatischen Widerstand aufweisen, oder Flusssensoren mit Heizdrähten eingesetzt werden. Das vom Differenzdrucksensor 13 gelieferte Signal kann als Einatemsignal und das vom Differenzdrucksensor 23 gelieferte Signal als Ausatemsignal bezeichnet werden. Die vom Prozessor berechnete Differenz von Einatemsignal minus Ausatemsignal wird als Atemflusssignal bezeichnet.

Die eingesetzten Flusssensoren müssen nur mit dem Medium Luft zurechtkommen, einen passenden Messbereich bis etwa 30 l/min aufweisen und einen hinreichend geringen Fließwiderstand haben. Da der Luftfluss in den Beatmungsschläuchen betragsmäßig größer als der durch Ein- und Ausatmen erzeugte Luftfluss ist, brauchen die eingesetzten Flusssensoren nicht einmal vorzeichenrichtig zu messen. So könnten Heizdrahtsensoren mit nur einem Heizdraht eingesetzt werden. Die Flusssensoren können im Beatmungsgerät 1 selbst untergebracht sein. Dann sind die Anschlüsse 44 und 54 als pneumatische Anschlüsse ausgeführt.

Um sicherzustellen, dass die Kennlinien der Flusssensoren 11 und 21 hinreichend gleich sind, können die Flusssensoren selektiert werden. Kostengünstiger ist es vermutlich, die Sensorsignale durch Software im eingebetteten Computer im Beatmungsgerät 1 so umzurechnen, dass die Kennlinien der Flusssensoren 11 und 21 gleich sind oder beide Kennlinien der Flusssensoren 11 und 21 eine Referenzkennlinie entsprechen, wobei die Referenzkennlinie mindestens in einer Beatmungsgeräteserie gleich ist.

In einer Weiterentwicklung der in Fig. 1 dargestellten Ausführungsform ist geplant, die beiden Strömungselemente 12 und 22 in das Y-Stück 7 zu integrieren. Ein zusätzlicher, entscheidender Nachteil der beatmungsgeräteinternen oder beatmungsgerätnahen Sensoren (vgl. Fig. 2) ist die fehlerhafte Volumenmessung, da die Beatmungsschlauchdehnbarkeit mit eingeht:

$$V_{Gesamt} = V_{Schlauch} + V_{Patient} \tag{4}$$

Das am Beatmungsgerät gemessene Atemvolumen $V_{Gesamt}$ ergibt sich als Summe der Volumenvergrößerung der Beatmungsschläuche $V_{Schlauch}$ und das tatsächliche Atemvolumen des Patienten $V_{Patient}$. Unter Verwendung von Gleichung (3) ergibt sich:

$$V_{Gesamt} = C_{Schlauch}\,\Delta P + C_{Patient}\,\Delta P \tag{5}$$

Da die Beatmungsschläuche eine vergleichsweise hohe Compliance $C_{schlauch}$ haben, also weich sind, wird das Atemvolumen des Patienten $V_{Patient}$ wesentlich kleiner als das am Beatmungsgerät gemessene Atemvolumen $V_{Gesamt}$ sein. Selbst eine rechnerische Kompensation funktioniert nur dann, wenn die Beatmungsschläuche bekannt sind und niemals andere als die vom Hersteller vorgegebenen Beatmungsschläuche verwendet werden.

Werden die beiden Strömungselemente 12 und 22 in das Y-Stück 7 integriert, können die Ein- und Ausatemschläuche 42 bzw. 52 entfallen. Die patientenseitigen Enden der Ein- und Ausatemschläuche 41 bzw. 51 werden direkt mit dem Y-Stück 7 verbunden. Allerdings erscheint es nicht geschickt, die zwei Flusswiderstände 12 und 22 in die beatmungsgerätseitigen Enden des Y-Stücks zu integrieren und anschließende beispielsweise durch die in Fig. 2 dargestellte pneumatische, analoge Brückenschaltung das Atemsignal des Patienten zu bestimmen. Geschickter ist es, den Atemfluss gleich im patientenseitigen Ende des Y-Stücks zu messen. Dort kann z. B. ein Flusswiderstand wie der Flusswiderstand 12 eingebaut werden. Der Druckabfall am Flusswiderstand kann über zwei Schläuche zu einem Differenzdrucksensor 31 im Beatmungsgerät 1 übertragen werden. Alternativ kann auch der Differenzdrucksensor 31 in das Y-Stück integriert werden oder gleich ein Flusssensor eingesetzt werden, der ein elektrisches Signal liefert wie beispielsweise eine mikromechanisch hergestellte Doppel-Hitzdrahtsonde. Das Y-Stück 7 kann ein MEMS oder MID (vgl. WO 2015/132239 A1) sein.

Bei allen Ausführungsformen ist darauf zu achten, dass an der Mimik der Patientenschnittstelle möglichst wenig Material angebracht wird.

Zusätzlich wird die Abdomenbewegung des Patienten 2 mit einer aus der EP 2 671 509 A1 bekannten Anordnung erfasst. Das Abdomenbewegungssignal A gemäß der EP 2 671 509 A1 hat einen streng monoton steigenden Zusammenhang mit dem Atemvolumen V. In Figur 1 ist ein elastisches Sensorteil 60 dargestellt, das mit einem Gurt 62 am Bauch des Patienten 2 befestigt ist. Der Druck im elastischen Sensorteil wird über den Druckschlauch 63 zum Beatmungsgerät 1 übertragen, dort in ein elektrisches Signal gewandelt, digitalisiert und dem eingebetteten Computer zugeführt. Über den Belüftungsschlauch 64 und ein nicht dargestelltes Ventil im Beatmungsgerät 1 kann das Beatmungsgerät Luft in das Sensorteil 60 hinein- oder aus dem Sensorteil 60 herausfließen lassen.

Dem Computer im Beatmungsgerät 1 steht also sowohl ein Atemflusssignal als auch ein Abdomenbewegungssignal A zur Steuerung der Beatmung zur Verfügung. Das Atemflusssignal ist insbesondere bei sich schnell ändernden Lecks unzuverlässig. Das Abdomenbewegungssignal ist aufgrund seines nichtlinearen Zusammenhangs mit dem Atemfluss nicht perfekt zur Steuerung des Beatmungsdrucks und/oder - flusses geeignet. Wenn man das Atemflusssignal zusammen mit dem Abdomenbewegungssignal zur Steuerung der Beatmung auswertet, kann jedes Signal die Schwächen des jeweils anderen Signals zumindest teilweise kompensieren.

In einer weiteren Ausführungsform kann bei dem Patienten 2 zusätzlich ein 3D-Beschleunigungssensor beispielsweise im Y-Stück 7, das auf der Stirn des Patienten 2 angebracht ist, eingesetzt werden. Zweck ist insbesondere, eine Änderung der Orientierung des Kopfes des Patienten 2 gegenüber der Erdbeschleunigung zu detektieren. Falls es eine Änderung gibt, ist die bisher aus dem Atemfluss zu und vom Patient 2 ermittelte Leckrate unzuverlässig. Deshalb wird in der Folge die Beatmung eher über die Druckkapsel gesteuert. Hält der Patient 2 seinen Kopf ruhig, ist umgekehrt die Leckrate konstant und kann aus dem Luftfluss bei Ein- und Ausatmung bestimmt werden. Solche 3D-Beschleunigungssensoren werden standardmäßig in Smartphones verbaut und sind deshalb billig.

Beispiele für den zeitlichen Verlauf des Atemvolumens V als auch des entsprechenden Atemflusssignals $\dot{V}$ finden sich in der WO 2005/09214 A1, Fig. 1 und Prof. Dr. med. Andreas Schulze, Zu GRUNDLAGEN und PRAXIS der BEATMUNG NEUGEBOHRENER, 2000, Andreas.Schulze@gyn.med.uni-muenchen.de, S. 15. Während der Inspiration ähnelt das Atemflusssignal $\dot{V}$ einer positiven Sinushalbwelle oder einem verschliffenen Rechteck oder Parallelogramm. Nach dem Wechsel zur Exspiration fällt das Atemflusssignal $\dot{V}$ schnell auf ein Minimum M (M<0) und steigt dann ähnlich einer gedämpften Exponentialfunktion $M \cdot \exp(-(t-T_M)/\tau)$ bis knapp unter die Nulllinie am Ende der Exspirationsphase an. Dabei ist t die Zeit, $T_M$ die Zeit ungefähr beim Minimum M und $\tau$ die Lungenzeitkonstante. Diese berechnet sich, wie in Prof. Dr. med. Andreas Schulze, Zu GRUNDLAGEN und PRAXIS der BEATMUNG NEUGEBOHRENER, 2000, Andreas.Schulze@gyn.med.uni-muenchen.de, S. 24 ausgeführt, als Produkt aus Lungencompliance und Atemwegswiderstand. Der Atemfluss am Ende der Exspiration beträgt etwa 1/5 bis 1/10 von M.

Während der Inspiration ist das Atemflusssignal $\dot{V}$ als auch die zeitliche Ableitung des Abdomenbewegungssignals $\dot{A}$ positiv. Während der Exspiration ist das Atemflusssignal $\dot{V}$ als auch die zeitliche Ableitung des Abdomenbewegungssignals $\dot{A}$ negativ.

Das Hauptziel hier besteht darin, eine falsche Erkennung der Inspiration zu vermeiden. Ein besonders kritischer Punkt ist die Ermittlung des Übergangs von Inspiration zu Exspiration. Der Übergang von Inspirationsmodus zu Exspirationsmodus im Beatmungsgerät erfolgt, nachdem sowohl das Atemflusssignal $\dot{V}$ als auch die zeitliche Ableitung des Abdomenbewegungssignals $\dot{A}$ vom Positiven kommend die Nulllinie unterschritten haben:

$$\dot{V}(\tau) < 0 \wedge \dot{A}(\tau - T_0) < 0 \qquad\qquad (6)$$

Entsprechend erfolgt der Übergang zwischen Exspirationsmodus und Inspirationsmodus im Beatmungsgerät, nach dem sowohl das Atemflusssignal $\dot{V}$ als auch die zeitliche Ableitung des Abdomenbewegungssignals $\dot{A}$ vom Negativen kommend die Nulllinie überschritten haben:

$$\dot{V}(\tau) > 0 \wedge \dot{A}(\tau - T_0) > 0 \qquad\qquad (7)$$

Erfahrungsgemäß ist das Abdomenbewegungssignal A etwas schneller als das Atemflusssignal $\dot{V}$. Deshalb kann man das Abdomenbewegungssignal um eine kurze positive Zeitdifferenz $T_0$ verzögern. $T_0$ kann aber auch 0 sein, sodass immer die aktuellen Messwerte ausgewertet werden.

Bisher wurde davon ausgegangen, dass das Abdomenbewegungssignal A einen streng monoton steigenden Zusammenhang mit dem Atemvolumen V hat. Wenn ein anderes Abdomenbewegungssignal C eingesetzt werden soll, muss es in ein Abdomenbewegungssignal umgerechnet werden, das einen streng monoton steigenden Zusammenhang mit dem Atemvolumen V hat, damit die Bedingungen (3) und (4) verwendet werden können. Alternativ können auch die Bedingungen (3) und (4) umgeformt werden und an das Abdomenbewegungssignal angepasst werden. Die DE 10 2012 014 141 A1 offenbart ein Abdomenbewegungssignal B, das einen streng monoton Zusammenhang zum Atemflusssignal $\dot{V}$ hat, wobei das Vorzeichen des Abdomenbewegungssignal B so gewählt wird, dass sich ein streng monoton steigender Zusammenhang ergibt. Aus Bedingung (3) ergibt sich somit als Bedingung für den Übergang von Inspirationsmodus zu Exspirationsmodus im Beatmungsgerät:

$$\dot{V}(\tau) < 0 \wedge B(\tau - T_0) < 0 \qquad (8)$$

Entsprechend ergibt sich aus Bedingung (4) als Bedingung für den Übergang von Exspirationsmodus zu Inspirationsmodus im Beatmungsgerät:

$$\dot{V}(\tau) > 0 \wedge B(\tau - T_0) > 0 \qquad (9)$$

Die bei dieser Ausführungsform ins Auge gefasste Beatmungsform ist PAV. Hierbei ergibt sich der Beatmungsdruck $P_i$ während der Inspiration zu:

$$P_i = P_{i0} + C_i \cdot V + R_i \cdot \dot{V} \qquad (10)$$

Dabei sind $P_{i0}$ eine Druckkonstante für die Inspiration, $C_i \cdot V$ der Druckanteil, der im Wesentlichen die Brustkorbelastizität (Compliance) kompensiert, sowie $R_i \cdot \dot{V}$ der Druckanteil, der im Wesentlichen Fließwiderstände in den Beatmungsschläuchen und den Bronchien kompensiert. $C_i$ und $R_i$ sind Konstanten, wobei insbesondere $C_i$ vom Patienten abhängt, also sinnvoll im Hinblick auf die Therapiebedürfnisse des Patienten gewählt werden muss. Der Druck während der Exspiration ergibt sich entsprechend zu:

$$P_e = P_{e0} + C_e \cdot V + R_e \cdot \dot{V} \qquad (11)$$

Hierbei ist $P_{e0}$ wieder eine Druckkonstante. Wird $P_{e0} = P_{i0} = CPAP$ gewählt, ergibt sich die in der DE 40 38 871 A1 offenbarten Beatmungsform. $C_e$ und $R_e$ sind Konstanten, die $C_i$ bzw. $R_i$ entsprechen und oft den gleichen Wert wie Ci bzw. $R_i$ haben.
Allgemein kann man die Beatmungsintensität $I_i$ während der Inspiration gemäß der Formel

$$I_i = I_{i0} + C_i \cdot V + R_i \cdot \dot{V} \qquad (12)$$

und die Beatmungsintensität $I_e$ während der Exspiration gemäß der Formel

$$I_e = I_{e0} + C_e \cdot V + R_e \cdot \dot{V} \qquad (13)$$

wählen, wobei $I_{i0}$ und $I_{eo}$ Konstanten sind. Wie oben erwähnt kann die Beatmungsintensität konkret ein Beatmungsdruck, ein Atemgasstrom eine Gebläsedrehzahl, ein Öffnungsquerschnitt oder ähnliches sein.
Figur 2 zeigt die Ausführungsform, die gerade zum Testen eingesetzt wird. Sie entspricht in etwa der in der DE 196 17 432 A1 offenbarten Anordnung. In dieser Ausführungsform ist nur ein Differenzdrucksensor 31 vorhanden, der gleich das Atemflusssignal liefert und im Beatmungsgerät 1 eingebaut ist. Die beatmungsgerätseitigen Anschlüsse der Flusssensoren 12 und 22 sind unmittelbar mit den Anschlüssen 43 bzw. 53 verbunden. An den patientenseitigen Anschlüssen der Flusssensoren 12 und 22 sind Beatmungsschläuche 42 bzw. 52 angeschlossen.
Jeder der Flusssensoren 12 und 22 weist einen beatmungsgerätseitigen und einen patientenseitigen pneumatischen Sensoranschluss auf. Die beatmungsgerätseitigen Sensoranschlüsse sind über gleiche Schläuche 34 und 35 über den positiven Sensoranschluss 38 mit dem positiven Eingang des Differenzdrucksensors 31 pneumatisch verbunden. Die patientenseitigen Sensoranschlüsse sind über gleiche Schläuche 32 und 33 über den negativen Sensoranschluss 39 mit dem negativen Eingang des Differenzdrucksensors 31 pneumatisch verbunden. So liefert der Differenzdrucksensor 31 bei Inspiration ein positives und bei Exspiration ein negatives Signal. Diese pneumatische Schaltung erledigt die Differenzbildung von Einatemsignal minus Ausatemsignal um das Atemflusssignal zu erhalten. Der Differenzdrucksensor 31 kann wie die Differenzdrucksensoren 13 und 23 analog oder digital sein und kann über elektrische Leitung 37 mit dem Computer im Beatmungsgerät 1 verbunden sein.
Die Schläuche 32, 33, 34 und 35 weisen einen deutlich geringeren Querschnitt als die Beatmungsschläuche 41, 42, 51 und 52 auf, so dass die Schläuche 32, 33, 34 und 35 als gleiche pneumatische Widerstände wirken und keinen pneumatischen Kurzschluss erzeugen. Die Gleichheit der Schläuche 32, 33, 34 und 35 bezieht sich sowohl auf den Querschnitt als auf deren Länge, sodass auch deren pneumatische Widerstand gleich ist.

In dieser Ausführungsform werden die Flusssensoren 12 und 22 selektiert, wodurch sich die Kennlinien der Flusssensoren um weniger als 2,5 % unterscheiden. Zusätzlich kann ein Offset-Abgleich und eine Kalibrierung im Betrieb erfolgen.

**Bezugszeichenliste**

| | |
|---|---|
| 1 | Beatmungsgerät |
| 2 | neonataler oder pädiatrischer Patient |
| 3 | Nasenmaske |
| 6 | Beatmungsschlauch |
| 7 | Y-Stück |
| 11, 21 | Flusssensor |
| 12, 22 | Strömungselement |
| 13, 23 | Differenzdrucksensor |
| 14, 24 | elektrische Leitung |
| 31 | Differenzdrucksensor |
| 32, 33, 34, 35 | gleiche Schläuche |
| 37 | elektrische Leitung |
| 38 | positiver Sensoranschluss |
| 39 | negativer Sensoranschluss |
| 41, 42 | Einatemschlauch |
| 43 | Einatemschlauchanschluss |
| 44 | elektrischer Anschluss |
| 51, 52 | Ausatemschlauch |
| 53 | Ausatemschlauchanschluss |
| 54 | elektrischer Anschluss |
| 60 | elastisches Sensorteil |
| 62 | Gurt |
| 63 | Druckschlauch |
| 64 | Belüfungsschlauch |
| 65 | Abdomenanschluss |
| 71 | Bedienelement |
| 72 | Bildschirm |

**Patentansprüche**

1. Beatmungsgerät (1) mit:

einem eingebetteten Computer mit einem Prozessor und einem nichtflüchtigen Speicher, wobei der nichtflüchtige Speicher eine Befehlsfolge speichert, die vom Prozessor im Betrieb ausgeführt wird;
einem Anschluss (43) für einen Einatemschlauch (41);
einem Anschluss (53) für einen Ausatemschlauch (51);
einem Sensoranschluss (44, 54; 38, 39) zum Anschließen einer Sensorleitung (14, 24; 37), wobei der Sensoranschluss mit dem Prozessor verbunden ist, um dem Prozessor ein Atemsignal zuzuführen, das aus dem Atemfluss eines Patienten (2) oder einer Testanordnung gewonnen wurde;
einem Abdomenanschluss (65) aufweist, der mit dem Prozessor verbunden ist, um dem Prozessor ein Abdomenbewegungssignal zuzuführen, wobei die Befehlsfolge sowohl das Abdomenbewegungssignal als auch das Atemsignal bei der Steuerung der Beatmung berücksichtigt,
wobei die Befehlsfolge den Prozessor veranlasst, zwischen einem Inspirationsmodus und einem Exspirationsmodus zu unterscheiden,
wobei der Prozessor vom Inspirationsmodus zum Exspirationsmodus wechselt, nachdem sowohl das Atemsignal als auch die zeitliche Ableitung des Abdomenbewegungssignals die Nulllinie unterschritten haben, wenn das Abdomenbewegungssignal streng monoton mit dem Atemvolumen ansteigt oder sowohl das Atemsignal als auch das Abdomenbewegungssignal die Nulllinie unterschritten haben, wenn das Abdomenbewegungssignal streng monoton mit dem Atemflusssignal ansteigt; und

wobei der Prozessor vom Exspirationsmodus zum Inspirationsmodus wechselt, nachdem sowohl das Atemsignal als auch die zeitliche Ableitung des Abdomenbewegungssignals die Nulllinie überschritten haben, wenn das Abdomenbewegungssignal streng monoton mit dem Atemvolumen ansteigt oder sowohl das Atemsignal als auch das Abdomenbewegungssignal die Nulllinie überschritten haben, wenn das Abdomenbewegungssignal streng monoton mit dem Atemflusssignal ansteigt.

2. Beatmungsgerät gemäß Anspruch 1, **dadurch gekennzeichnet, dass** die Befehlsfolge den Prozessor veranlasst, den Beatmungsdruck $P_i$ während eines Inspirationsmodus gemäß der Formel

$$P_i = P_{i0} + C_i \cdot V + R_i \cdot \dot{V}$$

und während eines Exspirationsmodus gemäß der Formel

$$P_e = P_{e0} + C_e \cdot V + R_e \cdot \dot{V}$$

einzustellen, wobei $P_{i0}$ und $P_{e0}$ Druckkonstanten sind, $C_i$, $R_i$, $C_e$ und $R_e$ Konstanten, $V$ das Atemvolumen und $\dot{V}$ das Atemsignal ist, wobei $\dot{V}$ bei der Inspiration positiv und $\dot{V}$ bei der Exspiration negativ ist.

3. Beatmungsgerät gemäß einem der vorangehenden Ansprüche, **dadurch gekennzeichnet, dass** der Sensoranschluss ein Einatemsensoranschluss (44) und die Sensorleitung eine Einatemsensorleitung (14) zu einem Einatemflusssensor (11, 12) zum Wandeln des Gasflusses im Einatemschlauch (41, 42) in ein Einatemsignal ist; wobei das Beatmungsgerät (1) ferner einen Ausatemsensoranschluss (54) für eine Ausatemsensorleitung (24) zu einem Ausatemflusssensor (21, 22) zum Wandeln des Gasflusses im Ausatemschlauch (51, 52) in ein Ausatemsignal aufweist, wobei der Ausatemsensoranschluss (54) mit dem Prozessor verbunden ist, um das Ausatemsignal dem Prozessor zuzuführen; wobei die Befehlsfolge Befehle enthält, die den Prozessor veranlassen, das Atemsignal als Differenz aus dem Einatemsignal minus Ausatemsignal zu berechnen.

4. Beatmungssystem mit einem Beatmungsgerät gemäß einem der Ansprüche 1 bis 2, **dadurch gekennzeichnet, dass** das Beatmungssystem ferner den Einatemschlauch (41, 42) mit einem in Serie geschalteten Einatemflusswiderstand (12) aufweist;

wobei das Beatmungssystem ferner den Ausatemschlauch (51, 52) mit einem in Serie geschalteten Ausatemflusswiderstand (22) aufweist;

wobei das Beatmungssystem ferner einen ersten Schlauch (32), einen zweiten Schlauch (33), einen dritten Schlauch (34) sowie einen vierten Schlauch (35) aufweist, wobei die pneumatischen Widerstände des ersten und zweiten Schlauchs (32, 33) gleich sind, wobei die pneumatischen Widerstände des dritten und vierten Schlauchs (34, 35) gleich sind, wobei die pneumatischen Widerstände des ersten bis vierten Schlauches (32, 33, 34, 35) größer als die pneumatischen Widerstände des Einatemschlauchs (41, 42) und des Ausatemschlauchs (51, 52) sind;

wobei das Beatmungssystem ferner einen Differenzdrucksensor (31) mit einem positiven und einem negativen Druckanschluss aufweist, wobei der erste Schlauch (32) den patientenseitigen Teil (42) des Einatemschlauchs mit dem negativen Eingang des Differenzdrucksensors (31) pneumatisch verbindet, wobei der zweite Schlauch (33) den patientenseitigen Teil (52) des Ausatemschlauchs mit dem negativen Eingang des Differenzdrucksensors (31) pneumatisch verbindet, wobei der dritte Schlauch (34) den geräteseitigen Teil (41) des Einatemschlauchs mit dem positiven Eingang des Differenzdrucksensors (31) pneumatisch verbindet, und wobei der vierte Schlauch (35) den geräteseitigen Teil (51) des Ausatemschlauchs mit dem positiven Eingang des Differenzdrucksensors (31) pneumatisch verbindet; wobei der elektrische Ausgang des Differenzdrucksensors das Atemsignal über eine elektrische Leitung (37) an den eingebetteten Computer liefert.

5. Steuer- und Testverfahren für ein Beatmungsgerät mit den Schritten:

Gewinnen eines Atemsignals (11, 21; 31) aus dem Atemfluss einer Testanordnung;

Zuführen des Atemsignals (11, 21; 31) zu einem Prozessor;

Gewinnen eines Abdomenbewegungssignals (62, 63, 64) aus der Testanordnung;

Zuführen (65) des Abdomenbewegungssignals (62, 63, 64) zu dem Prozessor;

Berechnen eines Beatmungsintensitätssignals in Abhängigkeit sowohl von dem Atemsignal als auch dem Abdomenbewegungssignal (62, 63, 64),

Wechseln von einem Inspirationsmodus zu einem Exspirationsmodus, nachdem sowohl das Atemsignal als auch die zeitliche Ableitung des Abdomenbewegungssignals negativ wird, wenn das Abdomenbewegungssignal streng monoton mit dem Atemvolumen ansteigt oder sowohl das Atemsignal als auch das Abdomenbewegungssignal negativ wird, wenn das Abdomenbewegungssignal streng monoton mit dem Atemflusssignal ansteigt; und

Wechseln vom Exspirationsmodus zum Inspirationsmodus, nachdem sowohl das Atemsignal als auch die zeitliche Ableitung des Abdomenbewegungssignals positiv wird, wenn das Abdomenbewegungssignal streng monoton mit dem Atemvolumen ansteigt oder sowohl das Atemsignal als auch das Abdomenbewegungssignal positiv wird, wenn das Abdomenbewegungssignal streng monoton mit dem Atemflusssignal ansteigt.

6. Steuer- und Testverfahren gemäß Anspruch 5, ferner **gekennzeichnet durch**:

Einstellen des Beatmungsdrucks $P_i$ während eines Inspirationsmodus gemäß der Formel

$$P_i = P_{i0} + C_i \cdot V + R_i \cdot \dot{V}$$

und während eines Exspirationsmodus gemäß der Formel

$$P_e = P_{e0} + C_e \cdot V + R_e \cdot \dot{V},$$

wobei $P_{i0}$ und $P_{e0}$ Druckkonstanten sind, $C_i$, $R_i$, $C_e$ und $R_e$ Konstanten, $V$ das Atemvolumen und $\dot{V}$ das Atemsignal ist, wobei $\dot{V}$ bei der Inspiration positiv und bei der Exspiration negativ ist.

7. Steuer- und Testverfahren gemäß Anspruch 5 oder 6, **dadurch gekennzeichnet, dass** das Atemsignal ein Einatemsignal ist und aus dem Gasfluss im Einatemschlauch (41, 42) gewonnen wird, wobei das Steuer- und Testverfahren ferner umfasst:

Gewinnen (11) eines Ausatemsignals aus dem Gasfluss im Ausatemschlauch (51, 52);

Berechnen des Atemsignals als Differenz aus dem Einatemsignal minus dem Ausatemsignal.

## Claims

1. An artificial respiration device (1) with:

an embedded computer with a processor and non-volatile memory, wherein the non-volatile memory stores a sequence of commands which is executed by the processor during operation;

a connector (43) for an inspiration tube (41);

a connector (53) for an expiration tube (51);

a sensor connector (44, 54; 38, 39) for the connection of a sensor cable (14, 24; 37), wherein the sensor connector is connected to the processor in order to provide a respiratory signal, which is obtained from a patient's airflow (2) or a test set-up, to the processor;

having an abdominal connector (65) which is connected to the processor in order to provide an abdominal movement signal to the processor, wherein the sequence of commands takes both the abdominal movement signal and the respiratory signal into consideration when controlling ventilation,

wherein the sequence of commands causes the processor to differentiate between inspiration mode and expiration mode,

wherein the processor switches from inspiration mode to expiration mode once both the respiratory signal and the time derivative of the abdominal movement signal have fallen below zero if the abdominal movement signal increases strictly monotonously with the respiratory volume or both the respiratory signal and the abdominal movement signal have fallen below zero if the abdominal movement signal increases strictly monotonously with the airflow signal; and

wherein the processor switches from expiration mode to inspiration mode once both the respiratory signal and the time derivative of the abdominal movement signal exceed zero if the abdominal movement signal increases strictly monotonously with the respiratory volume or both the respiratory signal and the abdominal movement signal exceed zero if the abdominal movement signal increases strictly monotonously with the airflow signal.

2. The artificial respiration device according to claim 1, **characterized in that** the sequence of commands causes the processor to adjust the ventilation pressure $P_i$ during inspiration mode according to the formula

$$P_i = P_{i0} + C_i \cdot V + R_i \cdot \dot{V}$$

and during expiration mode according to the formula

$$P_e = P_{e0} + C_e \cdot V + R_e \cdot \dot{V}$$

wherein $P_{i0}$ and $P_{e0}$ are pressure constants, $C_i$, $R_i$, $C_e$ and $R_e$ are constants,

$V$ is the respiratory volume and $\dot{V}$ is the respiratory signal, wherein $\dot{V}$ is positive during inspiration and $\dot{V}$ is negative during expiration.

3. The artificial respiration device according to any one of the preceding claims, **characterized in that** the sensor connector is an inspiration sensor connector (44) and the sensor cable is an inspiration sensor cable (14) to an inspiration flow sensor (11, 12) for conversion of the gas flow in the inspiration tube (41, 42) into an inspiration signal; wherein the artificial respiration device (1) furthermore

has a expiration sensor connector (54) for an expiration sensor cable (24) to an expiration flow sensor (21, 22) for conversion of the gas flow in the expiration tube (51, 52) into an expiration signal, wherein the expiration sensor connector (54) is connected to the processor in order to provide the expiration signal to the processor; wherein the sequence of commands includes commands which cause the processor to calculate the respiratory signal as the difference of the inspiration signal minus the expiration signal.

4. An artificial respiration system with an artificial respiration device according to any one of claims 1 to 2, **characterized in that** the artificial respiration system furthermore has an inspiration tube (41, 42) with a series-connected inspiratory flow resistor (12);

wherein the artificial respiration system furthermore has an expiration tube (51, 52) with a series-connected expiratory flow resistor (22);

wherein the artificial respiration system furthermore has a first tube (32), a second tube (33), a third tube (34) and a fourth tube (35), wherein the pneumatic resistances of the first and second tubes (32, 33) are equal, wherein the pneumatic resistances of the third and fourth tubes (34, 35) are equal, wherein the pneumatic resistances of the first to the fourth tubes (32, 33, 34, 35) are greater than the pneumatic resistances of the inspiration tube (41, 42) and the expiration tube (51, 52);

wherein the artificial respiration system furthermore has a differential pressure sensor (31) with a positive and a negative pressure connector, wherein the first tube (32) pneumatically connects the patient-side section (42) of the inspiration tube to the negative input of the differential pressure sensor (31), wherein the second tube (33) pneumatically connects the patient-side section (52) of the expiration tube to the negative input of the differential pressure sensor (31), wherein the third tube (34) pneumatically connects the device-side section (41) of the inspiration tube to the positive input of the differential pressure sensor (31), and wherein the fourth tube (35) pneumatically connects the device-side section (51) of the expiration tube to the positive input of the differential pressure sensor (31); wherein the electrical output of the differential pressure sensor provides the respiratory signal to the embedded computer via an electrical cable (37).

5. A control and test method for an artificial respiration device with the steps:

Obtaining a respiratory signal (11, 21; 31) from the airflow in a test set-up;

Providing the respiratory signal (11, 21; 31) to a processor;

Obtaining an abdominal movement signal (62, 63, 64) from the test set-up;

Providing (65) the abdominal movement signal (62, 63, 64) to the processor;

Calculating a ventilation intensity signal as a function of both the respiratory signal and the abdominal movement signal (62, 63, 64),

Switching from an inspiration mode to an expiration mode once both the respiratory signal and the time derivative of the abdominal movement signal have become negative if the abdominal movement signal increases strictly monotonously with the respiratory volume or both the respiratory signal and the abdominal movement signal have become negative if the abdominal movement signal increases strictly monotonously with the airflow signal; and

Switching from expiration mode to inspiration mode once both the respiratory signal and the time derivative of the abdominal movement signal have become positive if the abdominal movement signal increases strictly monotonously with the respiratory volume or both the respiratory signal and the abdominal movement signal have become positive if the abdominal movement signal increases strictly monotonously with the airflow signal.

6. The control and test method according to claim 5, further **characterized by**:

Adjustment of the ventilation pressure $P_i$ during inspiration mode according to the formula

$$P_i = P_{i0} + C_i \cdot V + R_i \cdot \dot{V}$$

and during expiration mode according to the formula

$$P_e = P_{e0} + C_e \cdot V + R_e \cdot \dot{V},$$

wherein $P_{i0}$ and $P_{e0}$ are pressure constants, $C_i$, $R_i$, $C_e$ and $R_e$ are constants,

V is the respiratory volume and $\dot{V}$ is the respiratory signal, wherein $\dot{V}$ is positive during inspiration and negative during expiration.

7. The control and test method according to claim 5 or 6, **characterized in that** the respiratory signal is an inspiration signal and is acquired from the airflow in the inspiration tube (41, 42), wherein the control and test method further includes:

Obtaining (11) an expiration signal from the airflow in the expiration tube (51, 52);

Calculating the respiratory signal as the difference of the inspiration signal minus the expiration signal.

**Revendications**

1. Appareil respiratoire (1) avec :

un ordinateur encastré équipé d'un processeur et d'une mémoire non volatile, la mémoire non volatile mémorisant une séquence de commandes exécutée par le processeur en situation de fonctionnement ;

un raccordement (43) pour un tuyau flexible d'inspiration (41) ;

un raccordement (53) pour un tuyau flexible d'expiration (51) ;

un raccordement de détecteur (44, 54 ; 38, 39) permettant de raccorder un câble de détecteur (14, 24; 37), le raccordement de détecteur étant relié au processeur pour amener au processeur un signal de respiration obtenu à partir du débit respiratoire d'un patient (2) ou d'un dispositif d'essai ;

un raccordement à l'abdomen (65) relié au processeur pour amener au processeur un signal de mouvement d'abdomen, la séquence de commandes prenant en compte tant le signal de mouvement d'abdomen que le signal de respiration lors de la commande de la respiration ;

la séquence de commandes amenant le processeur à établir la distinction entre un mode d'inspiration et un mode d'expiration ;

le processeur passant du mode d'inspiration au mode d'expiration après que tant le signal de respiration que la dérivée dans le temps du signal de mouvement d'abdomen passe en dessous de la ligne du zéro lorsque le signal de mouvement d'abdomen monte de façon fortement monotone avec le volume respiré ou que tant le signal de respiration que le signal de mouvement d'abdomen passent en dessous de la ligne du zéro lorsque le signal de mouvement d'abdomen monte de façon fortement monotone avec le signal de débit respiratoire ; et le processeur passant du mode d'expiration au mode d'inspiration après que tant le signal de respiration que la dérivée dans le temps du signal de mouvement d'abdomen passe au-dessus de la ligne du zéro lorsque le signal de mouvement d'abdomen monte de façon fortement monotone avec le volume respiré ou que tant le signal de respiration que le signal de mouvement d'abdomen passe au-dessus de la ligne du zéro lorsque le signal de mouvement d'abdomen monte de façon fortement monotone avec le signal de débit respiratoire.

2. Appareil respiratoire selon la revendication 1, **caractérisé en ce que** la séquence de commandes amène le processeur à régler la pression respiratoire $P_i$ pendant un mode d'inspiration suivant la formule :

$$P_i = P_{i0} + C_i \cdot V + R_i \cdot \dot{V}$$

et pendant un mode d'expiration suivant la formule :

$$P_e = P_{e0} + C_e \cdot V + R_e \cdot \dot{V}$$

$P_{i0}$ et $P_{e0}$ étant les constantes de pression, $C_i$, $R_i$, $C_e$ et $R_e$ étant des constantes, $V$ étant le volume respiré et $\dot{V}$ le signal de respiration, $\dot{V}$ étant positif lors de l'inspiration et $\dot{V}$ étant négatif lors de l'expiration.

3. Appareil respiratoire selon l'une quelconque des revendications précédentes, **caractérisé en ce que** le raccordement de détecteur est un raccordement de détecteur d'inspiration (44) et que le câble de détecteur est un câble de détecteur d'inspiration (14) conduisant à un détecteur de débit d'inspiration (11, 12) en vue de convertir le débit gazeux dans le tuyau flexible d'inspiration (41, 42) en un signal d'inspiration ; l'appareil respiratoire (1) comportant en outre :

un raccordement de détecteur d'expiration (54) pour un câble de détecteur d'expiration (24) conduisant à un détecteur de débit d'expiration (21, 22) en vue de convertir le débit gazeux dans le tuyau flexible d'expiration (51, 52) en signal d'expiration, le raccordement de détecteur d'expiration (54) étant relié au processeur pour amener le signal d'expiration au processeur ; la séquence de commandes contenant des commandes permettant au processeur de calculer le signal de respiration sous la forme de la différence entre le signal d'inspiration auquel on retire le signal d'expiration.

4. Système respiratoire équipé d'un appareil respiratoire selon l'une quelconque des revendications 1 à 2, **caractérisé en ce que** le système respiratoire comporte en outre le tuyau flexible d'inspiration (41, 42) avec une résistance de débit d'inspiration (12) raccordée en série ;
le système respiratoire comportant en outre le tuyau flexible d'expiration (51, 52) avec une résistance de débit d'expiration (22) raccordée en série ;
le système respiratoire comportant en outre un premier tuyau flexible (32), un deuxième tuyau flexible (33), un troisième tuyau flexible (34) ainsi qu'un quatrième tuyau flexible (35), les résistances pneumatiques du premier et deuxième tuyau flexible (32, 33) étant identiques, les résistances pneumatiques du troisième et quatrième tuyau flexible (34, 35) étant identiques, les résistances pneumatiques du premier au quatrième tuyau flexible (32, 33, 34, 35) étant plus importantes que les résistances pneumatiques du tuyau flexible d'inspiration (41, 42) et du tuyau flexible d'expiration (51, 52) ;
le système respiratoire comportant en outre un détecteur de pression différentielle (31) avec un raccordement de pression positif et négatif, le premier tuyau flexible (32) reliant de façon pneumatique la partie du côté patient (42) du tuyau flexible d'inspiration à l'entrée négative du détecteur de pression différentielle (31), le deuxième tuyau flexible (33) reliant de façon pneumatique la partie du côté patient (52) du tuyau flexible d'expiration à l'entrée négative du détecteur de pression différentielle (31), le troisième tuyau flexible (34) reliant de façon pneumatique la partie côté appareil (41) du tuyau flexible d'inspiration à l'entrée positive du détecteur de pression différentielle (31) et le quatrième tuyau flexible (35) reliant de façon pneumatique la partie côté appareil (51) du tuyau flexible d'expiration à l'entrée positive du détecteur de pression différentielle (31) ; la sortie électrique du détecteur de pression différentielle amenant le signal de respiration à l'ordinateur encastré en passant par un câble électrique (37).

5. Procédé de commande et d'essai pour un appareil respiratoire avec les étapes suivantes :

obtention d'un signal de respiration (11, 21 ; 31) à partir du débit respiratoire d'un dispositif d'essai ;
amenée du signal de respiration (11, 21 ; 31) à un processeur ;
obtention d'un signal de mouvement d'abdomen (62, 63, 64) provenant du dispositif d'essai ;
amenée (65) du signal de mouvement d'abdomen (62, 63, 64) jusqu'au processeur ;
calcul d'un signal d'intensité respiratoire à partir tant du signal de respiration que du signal de mouvement d'abdomen (62, 63, 64) ;
passage d'un mode d'inspiration à un mode d'expiration après que tant le signal de respiration et la dérivée dans le temps du signal de mouvement d'abdomen soient négatifs lorsque le signal de mouvement d'abdomen monte de façon fortement monotone avec le volume respiré ou que tant le signal de respiration que le signal de mouvement d'abdomen soient négatifs lorsque le signal de mouvement d'abdomen monte de façon fortement monotone avec le signal de débit respiratoire ; et
passage du mode d'expiration au mode d'inspiration après que tant le signal de respiration que la dérivée dans le temps du signal de mouvement d'abdomen soient positifs lorsque le signal de mouvement d'abdomen monte de façon fortement monotone avec le volume respiré ou tant le signal de respiration que le signal de mouvement d'abdomen soient positifs lorsque le signal de mouvement d'abdomen monte de façon fortement monotone avec le signal de débit respiratoire.

6. Procédé de commande et d'essai selon la revendication 5, **caractérisé en outre par**:
le réglage de la pression respiratoire $P_i$ pendant un mode d'inspiration suivant la formule :

$$P_i = P_{i0} + C_i \cdot V + R_i \cdot \dot{V}$$

et pendant un mode d'expiration suivant la formule :

$$P_e = P_{e0} + C_e \cdot V + R_e \cdot \dot{V},$$

$P_{i0}$ et $P_{e0}$ étant des constantes de pression, $C_i$, $R_i$, $C_e$ et $R_e$ étant des

constantes, V étant le volume respiré et $\dot{V}$ étant le signal de respiration, $\dot{V}$ étant négatif lors de l'inspiration et positif lors de l'expiration.

7. Procédé de commande et d'essai selon la revendication 5 ou 6, **caractérisé en ce que** le signal de respiration est un signal d'inspiration et est obtenu à partir du débit gazeux dans le tuyau flexible d'inspiration (41, 42), le procédé de commande et d'essai comprenant en outre :

l'obtention (11) d'un signal d'expiration provenant du débit gazeux dans le tuyau flexible d'expiration (51, 52) ;
le calcul du signal de respiration comme la différence du signal d'inspiration auquel on soustrait le signal d'expiration.

FIG. 2

FIG. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 112010001519 B4
- DE 10023473 A1
- DE 102012014141 A1
- DE 102007035549 A1
- EP 2671509 A1
- DE 19617432 A1
- DE 102010000219 A1
- DE 69924163 T2
- EP 1382294 B1
- DE 69922511 T2
- DE 4038871 A1
- WO 2015132239 A1
- WO 200509214 A1

**In der Beschreibung aufgeführte Nicht-Patentliteratur**

- **PROF. DR. MED. ANDREAS SCHULZE.** *Zu GRUNDLAGEN und PRAXIS der BEATMUNG NEU-GEBOHRENER,* 2000